# EUROPEAN PATENT SPECIFICATION

(11) **EP 3 216 454 B1**
(45) Date of publication and mention of the grant of the patent: **24.08.2022**
(21) Application number: 16177331.2
(22) Date of filing: 30.06.2016
(51) Int. Cl.: A61K 31/715, A61P 31/16

(54) **COMPOSITION COMPRISING POLYSACCHARIDES EXTRACTED FROM LENTINUS, PORIA AND TREMELLA AND USE THEREOF FOR COMBATING INFLUENZA VIRUS**
ZUSAMMENSETZUNG ENTHALTEND AUS LENTINUS, PORIA UND TREMELLA EXTRAHIERTE POLYSACCHARIDE UND DEREN VERWENDUNG ZUR BEKÄMPFUNG DES INFLUENZA-VIRUS
COMPOSITION RENFERMANT DES POLYSACCHARIDES EXTRAITS DE LENTINUS, PORIA ET TREMELLA ET UTILISATION DE CELLE-CI POUR LUTTER CONTRE LE VIRUS DE L'INFLUENZA

(30) Priority: 10.03.2016 CN 201610135683
(43) Date of publication of application: 13.09.2017
(73) Proprietor: Infinitus (China) Company Ltd., Jiang Men City, Guangdong 529100 (CN)
(72) Inventor: HU, Minghua, Jiang Men City, Guangdong 529100 (CN); MA, Chung Wah, Jiang Men City, Guangdong 529100 (CN); MA, Fangli, Jiang Men City, Guangdong 529100 (CN); GUO, Xiaolei, Jiang Men City, Guangdong 529100 (CN); LUO, Zhen, Jiang Men City, Guangdong 529100 (CN); YIN, Xiquan, Jiang Men City, Guangdong 529100 (CN)
(74) Representative: Hollah, Dorothee

(56) References cited:
- IRINODA K ET AL: "Chemiluminescence and cytokine responses of influenza virus-infected mice pretreated with immunomodulator lentinan/Protection against aerogenic influenza virus infection in mice by polysaccharide lentinan", INTERNATIONAL JOURNAL OF IMMUNOPHARMACOLOGY, ELMSFORD,NY, US, vol. 13, no. 6, 1 January 1991 (1991-01-01), page 701, XP025851209, ISSN: 0192-0561, DOI: 10.1016/0192-0561(91)90184-9 [retrieved on 1991-01-01]
- IRINODA K ET AL: "Stimulation of microbicidal host defence mechanisms against aerosol influenza virus infection by lentinan", INTERNATIONAL JOURNAL OF IMMUNOPHARMACOLOGY AUG 1992, vol. 14, no. 6, August 1992 (1992-08), pages 971-977, XP025491802, ELMSFORD, NY, US ISSN: 0192-0561, DOI: 10.1016/0192-0561(92)90140-G
- XIE G ET AL: "Enhanced Immune Effects of Pachymaran on Inactivated Influenza Virus Vaccine", LIFE SCIENCE RESEARCH, vol. 13, no. 3, 30 June 2009 (2009-06-30), pages 246-250, XP002769609, ISSN: 1007-7847
- LI S ET AL: "Adjuvant effect of total polysaccharides from poria cocos on H1N1 influenza and hbsag antigens", CHINESE JOURNAL OF PHARMACOLOGY AND TOXICOLOGY, vol. 29, no. 1, 1 February 2015 (2015-02-01), pages 60-64, XP002769610, ISSN: 1000-3002, DOI: 10.3867/j.issn.1000-3002.2015.01.010
- LIN Z-B ET AL: "Effect of tremella polysaccharide on immune function depressed by cyclophosphamide, physical stress and aging in mice", EUROPEAN JOURNAL OF PHARMACOLOGY, ELSEVIER SCIENCE, NL, vol. 183, no. 3, 3 July 1990 (1990-07-03), page 900, XP023837911, ISSN: 0014-2999, DOI: 10.1016/0014-2999(90)92729-3 [retrieved on 1990-07-03]
- GAO Q ET AL: "Characterisation of acidic heteroglycans from Tremella fuciformis Berk with cytokine stimulating activity", CARBOHYDRATE RESEARCH, PERGAMON, GB, vol. 288, 19 July 1996 (1996-07-19), pages 135-142, XP004018883, ISSN: 0008-6215, DOI: 10.1016/0008-6215(96)00091-2
- CUI J L; LIN Z B: "Effects of Tremella Polysaccharide on Immune Function of Physically-Stressed Mice", JOURNAL OF CHINESE PHARMACEUTICAL SCIENCES, vol. 1, no. 2, 1992, pages 49-53, XP002769611, ISSN: 1003-1057
- WANG Q, HU M H, DONG Y ET AL: "The effect of pachymaran on the change in lymphocyte subsets induced by cyclophosphamide", CHINESE JOURNAL OF IMMUNOLOGY, vol. 27, no. 3, 2011, pages 228-231, XP002769612, ISSN: 1000-484X
- PENG X P ET AL: "Effect of pachymaran on humoral immune function in immunosuppressed mice induced by cyclophosphamide", PHARMACOLOGY AND CLINICS OF CHINESE MATERIA MEDICA, vol. 29, no. 5, 2013, pages 69-72, XP002769613, ISSN: 1001-859X
- HAMURO J ET AL: "The significance of the higher structure of the polysaccharides lentinan and pachymaran with regard to their antitumour activity", CHEMICO-BIOLOGICAL INTERACTIONS, ELSEVIER SCIENCE IRLAND, IR, vol. 3, no. 1, 1 February 1971 (1971-02-01), pages 69-71, XP023813010, ISSN: 0009-2797, DOI: 10.1016/0009-2797(71)90026-3 [retrieved on 1971-02-01]
- HOFFMANN G C ET AL: "Structure and molecular size of pachyman", CARBOHYDRATE RESEARCH, PERGAMON, GB, vol. 20, no. 1, 1 November 1971 (1971-11-01), pages 185-188, XP026619131, ISSN: 0008-6215, DOI: 10.1016/S0008-6215(00)84962-9 [retrieved on 1971-11-01]
- LUO X ET AL: "Effects of compound polysaccharide on cellular immunity in mice", CHINESE JOURNAL OF FOOD HYGIENE, vol. 28, no. 2, February 2016 (2016-02), pages 186-191, XP009510032,
- Jiang Pi ET AL: "Immunomodulatory effects of polysaccharide compounds in macrophages revealed by high resolution AFM : Immunomodulatory effects of polysaccharide compounds", SCANNING., vol. 38, no. 6, 9 June 2016 (2016-06-09), pages 792-801, XP055534739, US ISSN: 0161-0457, DOI: 10.1002/sca.21329

## Description

### FIELD OF THE INVENTION

The present invention relates to polysaccharide composition consisting of lentinan, pachymaran and tremella polysaccharide and use thereof.

### BACKGROUD OF THE INVENTION

Seasonal influenza (abbreviated as flu) is a common acute respiratory infectious disease caused by influenza virus, and is epidemic in the crowd year after year. The crowd generally has resistance, with mild symptoms and low mortality. After suffering from the flu, the elderly, children, or those with certain underlying diseases or weak physique are susceptible to severe complications such as pneumonia, bronchitis, congestive heart failure, gastroenteritis, syncope, hallucination, etc. The consequences are very serious, and sometimes complications even cause death. According to the statistics of the World Health Organization (WHO), the epidemic of seasonal influenza causes 3-5 million severe cases and 250,000-500,000 deaths every year.

In early 2009, an epidemic situation of a respiratory disease began to appear in Mexico, which is an epidemic caused by a new human influenza A virus, i.e. H1N1 influenza A virus. The epidemic situation was later proved to be caused by a new swine-derived influenza virus A(H1N1)-(S-OIV), and was referred to as "human infection with swine influenza". With gradual deepening of the research and the continuous knowledge of the disease, it was renamed as "H1N1 influenza A" by the WHO. Meanwhile, the epidemic of this virus was defined as an international public health event. In comparison with the seasonal influenza virus, fulminant influenza viruses such as the H1N1 influenza A virus and H7N9 influenza virus are more detrimental, and can cause a worldwide panic.

Polysaccharide is formed by condensation and dehydration of a plurality of monosaccharide molecules, and is a category of carbohydrate substances with complicated and huge molecule structures. Polysaccharide is a type of immunomodulator, and many naturally occurring plant polysaccharides have modulating effects on the immune system. The immune system of human body is closely associated with anti-virus infection. Firstly, 95% or more of pathogenic infections occur at the mucosa or cause diseases via mucosal invasion. The mucosa of the respiratory tract is not only the infection site of influenza viruses, but also the site where the host defends against the viral infection. There are plenty of immune cells and immune molecules on the respiratory tract mucosa, which play very important role in anti-virus immune defense. Secondly, all immune cells such as macrophages, NK cells, T lymphocytes, B lymphocytes, etc. and cytokines such as IFN-γ, TNF-α, etc. in the body play important protective roles in anti-virus infection. Therefore, it is feasible to some extent to screen active substances with anti-virus effects from polysaccharides of Chinese herbal medicine.

Current studies show that polysaccharides have significant immunomodulatory effects, but anti-virus effects thereof are less reported. The value of polysaccharides in anti-virus application needs to be further developed.

### SUMMARY OF THE INVENTION

In view of this, the technical problem to be solved by the present invention is to provide a polysaccharide composition and use thereof. The composition provided in the present invention has good anti-virus effects.

The composition as provided in the present invention consists of lentinan, pachymaran and tremellan polysaccharide;

wherein the mass ratio of lentinan, pachymaran and tremellan is (1-3):(1-3):(1-3).

In the examples of the present invention, the mass ratio of lentinan, pachymaran and tremellan is (1-3):(1-2):(1-2).

In the examples of the present invention, the mass ratio of lentinan, pachymaran and tremellan is (2-3):1:1.

In the examples of the present invention, the mass ratio of lentinan, pachymaran and tremellan is (1-2):1:1.

In the examples of the present invention, the mass ratio of lentinan, pachymaran and tremellan is 1:(1-3):(1-3).

In some examples, the mass ratio of lentinan, pachymaran and tremellan is 1:1:1.

In some examples, the mass ratio of lentinan, pachymaran and tremellan is 1:3:3.

In some examples, the mass ratio of lentinan, pachymaran and tremellan is 3:1:1.

In some examples, the mass ratio of lentinan, pachymaran and tremellan is 1:3:1.

In some examples, the mass ratio of lentinan, pachymaran and tremellan is 1:2:3.

In some examples, the mass ratio of lentinan, pachymaran and tremellan is 2:1:3.

In some examples, the mass ratio of lentinan, pachymaran and tremellan is 3:2:1.

Polysaccharides are very abundant in plants. They are soluble in water, and are generally prepared by water extraction and alcohol precipitation. In addition, assisted extraction of polysaccharides in plants is also performed in the prior art with ultrasonic wave, microwave, enzymolysis and other means.

Lentinan is extracted from the fruiting body of shiitake. Clinical and pharmacological studies have shown that lentinan has anti-tumor effect, immunomodulatory effect, interferon formation stimulating effect, etc.

Pachymaran is extracted from the sclerotium of tuckahoe, a fungus of family Polyporaceae. It has immune-enhancing activity, and can be used for anti-virus, anti-tumor, reduction of side effects of radiotherapy and chemotherapy, treatment of chronic hepatitis, as well as anti-aging, etc.

Tremellan is extracted from the fruiting body of tremella. Currently, tremellan is clinically used for treatment of leukopenia caused by chemotherapy or radiotherapy of tumer and leukopenia caused by other reasons. In addition, it can also be used for treating chronic bronchitis.

In the present invention, lentinan, pachymaran and tremellan are compounded. The resultant composition can significantly improve the survival rate and quality of life of mice infected by influenza virus, improve the pulmonary inflammatory lesion degree in mice infected by influenza virus, significantly improve levels of specific antibodies in serum of mice infected by influenza virus, significantly decrease the virus content in the lung of mice infected by influenza virus, as well as improve levels of cytokines IL-2 and IL-5 in the lung. In comparison with existing vaccines or single polysaccharides, the polysaccharide composition as provided in the present invention has more significant protective effects on mice infected by influenza virus.

The lentinan, pachymaran and tremellan employed in the present invention can be self-made or commercially purchased. Implementations with lentinan, pachymaran and tremellan from any source are within the protection scope of the present application. In the present invention, the lentinan, pachymaran, tremellan or polysaccharide compositions employed in the experiments were all provided by Infinitus (China) Company Ltd.

The present invention provides the polysaccharide composition as provided in the present invention for use as a medicament (also referred to as health care product), particularly for use in the treatment of influenza

According to the mode for producing cytokines and biological functions, T cells are divided into 2 types of very different subgroups, which are referred to as Th1 and Th2, respectively. Th1 cells mainly secrete cytokines such as IL-2, IL-12, IFN-γ and TNF-β, etc., and play an important role in combating inflammatory responses caused by intracellular microbial infection and cellular immunity through promoting activation and proliferation of cytotoxic T cell (Tc), natural killer cell (NK) and microphage, mediating the immune response associated with cytotoxicity and local inflammation, and participating in cellular immunity and delayed type hypersensitivity. Th2 cells mainly secrete cytokines such as IL-4, IL-5, IL-6, IL-10, etc.. Their main function is to stimulate the proliferation of B cells to produce antibodies, participate in humoral immunity, and play a certain role in immunity against infection of extracellular microorganism or parasite as well as in the development of allergy. Therefore, cytokines such as IL-2, IL-5, etc. play important roles in protection against viruses in the body. It is found that the polysaccharide composition as provided in the present invention can significantly improve levels of IL-2 and IL-5 in mice infected by viruses, which suggests that the polysaccharide composition can play a role in anti-virus by regulating the differentiation level of T cells in the body.

The present invention provides the polysaccharide composition as provided in the present invention for use as an anti-influenza virus medicament (also referred to as health care product). Also provided is the use of the polysaccharide composition as a food.

Influenza is an acute upper respiratory tract infection caused by influenza virus, and can cause acute lung injury and severe complications. Studies have shown that excessive immune response is one of the major factors causing pathological damage. Due to enlargement of lung volume caused by congestion of the alveolar capillary, enlargement of the alveolar septum and interstitial edema after virus infection, lung indexes are commonly used as important indicators for evaluating the anti-virus efficacy. The present experiments found that, after using the polysaccharide compositions as provided in the present invention, the lung surface of mice infected by virus appeared reddish with no ecchymosis or petechiae, had no obvious areas of lung consolidation, and very few lung lobes exhibited the phenomena of congestion. The lung indexes were significantly decreased as compared to both the blank group and the vaccine group, suggesting that the polysaccharide compositions had the effect of improving the lung damage caused by virus infection.

In the examples of the present invention, the influenza virus is seasonal influenza virus or influenza A virus.

As a preference, the influenza virus is seasonal influenza virus (A/PR/8(H1N1)) or H1N1 influenza A virus (Mexican pandemic flu H1N1).

The human dosage of the polysaccharide composition as provided in the present invention for combating influenza virus is 0.1-1 g/day, preferably 0.5-0.8 g/day.

The polysaccharide composition as provided in the present invention may be comprised in a food* and/or medicament* (also referred to as health care product). *Not belonging to the present invention

The food comprises the polysaccharide composition as provided in the present invention and ingredients acceptable in food.

The medicament comprises the polysaccharide composition as provided in the present invention and pharmaceutically acceptable excipients.

As a preference, the medicament is an oral formulation. Preferably, the dosage form of the oral formulation is tablet, capsule, pill, granule, decoction, paste, distillate medicinal water, oral liquid, dropping pill or syrup.

The anti-influenza virus health care product comprises the polysaccharide composition as provided in the present invention and excipients acceptable in health care product.

As a preference, the health care product is an oral formulation. Preferably, the dosage form of the oral formulation is tablet, capsule, pill, granule, decoction, paste, distillate medicinal water, oral liquid, dropping pill or syrup.

In the present invention, lentinan, pachymaran and tremellan are compounded. The resultant composition can significantly improve the survival rate and quality of life of mice infected by influenza virus, improve the pulmonary inflammatory lesion degree in mice infected by influenza virus, significantly improve levels of specific antibodies in serum of mice infected by influenza virus, significantly decrease the virus content in the lung of mice infected by influenza virus, as well as improve levels of cytokines IL-2 and IL-5 in the lung. In comparison with existing vaccines or single polysaccharides, the polysaccharide composition as provided in the present invention has more significant protective effects on mice infected by influenza virus.

### DESCRIPTION OF THE DRAWINGS

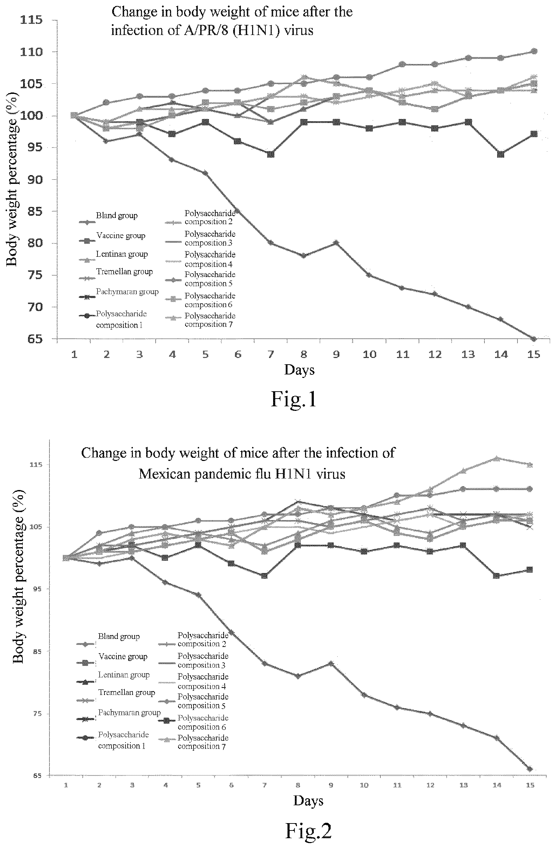

Figure 1 shows the change of mice body weight after infection by A/PR/8 (H1N1) virus.
Figure 2 shows the change of mice body weight after infection by Mexican pandemic flu H1N1 virus.

### DETAILED EMBODIMENTS

The present invention provides a polysaccharide composition and use thereof, which can be achieved by appropriately improving the process parameters by those skilled in the art in light of the present disclosure.

The reagents, medicaments, animals or instruments are all commonly commercial available, and can be commercially purchased.

Wherein, lentinan, pachymaran and tremellan were commercially purchased.

The present invention is further illustrated in conjunction with the following examples:

### Example 1

Lentinan 10 g, pachymaran 10 g and tremellan 10 g were mixed to obtain polysaccharide composition 1.

### Example 2

Lentinan 10 g, pachymaran 30 g and tremellan 30 g were mixed to obtain polysaccharide composition 2.

### Example 3

Lentinan 30 g, pachymaran 10 g and tremellan 10 g were mixed to obtain polysaccharide composition 3.

### Example 4

Lentinan 10 g, pachymaran 30 g and tremellan 10 g were mixed to obtain polysaccharide composition 4.

### Example 5

Lentinan 10 g, pachymaran 20 g and tremellan 30 g were mixed to obtain polysaccharide composition 5.

### Example 6

Lentinan 20 g, pachymaran 10 g and tremellan 30 g were mixed to obtain polysaccharide composition 6.

### Example 7

Lentinan 30 g, pachymaran 20 g and tremellan 10 g were mixed to obtain polysaccharide composition 7.

### Experimental example 1

### 1. Experimental animals

BALB/c mice (female, 6~8 weeks old, body weight 18~22 g) were provided by Beijing HFK Bioscience Co. Ltd., with license number: SCXK (Jing) 2014-0004. They were housed in the State Key Laboratory of Agricultural Microbiology, Huazhong Agricultural University. The license number for using experimental animals is: HZAUMU2015-0006.

### 2. Main reagents

Mice lung adapted strain of influenza virus: A/PR/8 (H1N1), concentration: 10⁴ PFU/ml. Mexican pandemic flu H1N1, concentration: 10⁴ PFU/ml.

Influenza virus vaccine contains 10⁴ PFU/ml inactivated virus and mixed MF59 adjuvant at equal amount.

Influenza virus and vaccine were both provided by the State Key Laboratory of Agricultural Microbiology, Huazhong Agricultural University.

Normal saline was from Anhui Global Pharmaceutical Co. Ltd.

### 3. Test samples

Lentinan, pachymaran, tremellan and polysaccharide compositions prepared in Examples 1~7 were respectively formulated into 20 mg/mL polysaccharide solutions. The dosage for intragastrical administration is 200 mg/kg (100 µl/10g based on the body weight of the mouse).

### 4. Experimental method

The function of random number of Excel software was employed to randomly divide BALB/c mice into blank group, vaccine group, lentinan group, pachymaran group, tremellan group, polysaccharide composition 1 (prepared in Example 1), polysaccharide composition 2 (prepared in Example 2), polysaccharide composition 3 (prepared in Example 3), polysaccharide composition 4 (prepared in Example 4), polysaccharide composition 5 (prepared in Example 5), polysaccharide composition 6 (prepared in Example 6), and polysaccharide composition 7 (prepared in Example 7); with 17 mice in each group. They were respectively operated according to the following processes:

Blank group: intragastrically administrated with normal saline for 30 days.

Vaccine group: intragastrically administrated with normal saline for 30 days, and 100 µl of influenza virus vaccine was intramuscularly injected on Day 10 and Day 25, respectively.

Lentinan group, pachymaran group, tremellan group, polysaccharide composition groups 1~7: intragastrically administrated with 200 mg/kg polysaccharide for 30 days, and 100 µl influenza virus vaccine (containing 10⁴ PFU/ml of inactivated virus and mixed adjuvant at equal amount) was intramuscularly injected on Day 10 and Day 25, respectively.

On Day 31 (Day 0), 3 mice were taken from each group, from which blood and lungs were collected.

On Day 32, each group was infected by 50 µl H1N1 influenza virus through nasal dropping.

From Day 33 to Day 46 (within 2 weeks after infection), the change in body weight of test mice was weighed everyday (Table 1), the survival state of mice was observed, and statistical analysis was conducted for mortality (Table 2). On day 46 (Day 14 after infection), the experiment was completed.

**Table 1. Change in body weight of mice after A/PR/8 (H1N1) virus infection (n=8, x̅±S)**

| Groups | Change in body weight (%) | | | | | | | | | | | | | |
|---|---|---|---|---|---|---|---|---|---|---|---|---|---|---|
| | Day 1 | Day 2 | Day 3 | Day 4 | Day 5 | Day 6 | Day 7 | Day 8 | Day 9 | Day 10 | Day 11 | Day 12 | Day 13 | Day 14 |
| Blank group | 100 | 96 | 97 | 93 | 91 | 85 | 80 | 78 | 80 | 75 | 73 | 72 | 70 | 68 |
| Vaccine group | 100 | 98 | 99 | 97 | 99 | 96 | 94 | 99 | 99 | 98 | 99 | 98 | 99 | 98 |
| Lentinan group | 100 | 99 | 101 | 102 | 101 | 100 | 99 | 101 | 103 | 104 | 102 | 101 | 103 | 104 |
| Tremellan group | 100 | 98 | 99 | 100 | 101 | 102 | 103 | 106 | 105 | 104 | 103 | 104 | 104 | 104 |
| Pachymaran group | 100 | 99 | 99 | 100 | 101 | 102 | 103 | 103 | 102 | 103 | 104 | 105 | 103 | 104 |
| Polysaccharide composition 1 | 100 | 102 | 103 | 103 | 104 | 104 | 105 | 105 | 106 | 106 * | 108 * | 108 * | 109 * | 109 * |
| Polysaccharide composition 2 | 100 | 99 | 99 | 100 | 101 | 102 | 99 | 101 | 103 | 104 | 102 | 101 | 103 | 104 |
| Polysaccharide composition 3 | 100 | 99 | 101 | 102 | 101 | 100 | 103 | 106 | 105 | 104 | 103 | 104 | 104 | 104 |
| Polysaccharide composition 4 | 100 | 98 | 99 | 100 | 101 | 102 | 103 | 103 | 102 | 103 | 104 | 105 | 103 | 104 |
| Polysaccharide composition 5 | 100 | 99 | 99 | 100 | 101 | 102 | 99 | 101 | 103 | 104 | 102 | 101 | 103 | 104 |
| Polysaccharide composition 6 | 100 | 98 | 98 | 100 | 102 | 102 | 101 | 102 | 103 | 104 | 102 | 101 | 103 | 104 |
| Polysaccharide composition 7 | 100 | 99 | 101 | 101 | 101 | 102 | 103 | 106 | 105 | 104 | 103 | 104 | 104 | 104 |

| | | | | | | | | | | | | | | |
|---|---|---|---|---|---|---|---|---|---|---|---|---|---|---|
| In comparison with the vaccine group, **P*<0.05, ***P*<0.01. | | | | | | | | | | | | | | |

**Table 2. Comparison of survival rates of mice among various groups after A/PR/8 (H1N1) virus infection (n=8)**

| Groups | Survival rates (%) | | | | | | | | | | | | | |
|---|---|---|---|---|---|---|---|---|---|---|---|---|---|---|
| | Day 1 | Day 2 | Day 3 | Day 4 | Day 5 | Day 6 | Day 7 | Day 8 | Day 9 | Day 10 | Day 11 | Day 12 | Day 13 | Day 14 |
| Blank group | 100 | 100 | 100 | 100 | 100 | 100 | 87.5 | 87.5 | 87.5 | 75 | 62.5 | 50 | 37.5 | 37.5 |
| Vaccine group | 100 | 100 | 100 | 100 | 100 | 100 | 100 | 100 | 100 | 87.5 | 87.5 | 87.5 | 87.5 | 87.5 |
| Lentinan group | 100 | 100 | 100 | 100 | 100 | 100 | 100 | 100 | 100 | 100 | 100 | 100 | 100 | 100 |
| Tremellan group | 100 | 100 | 100 | 100 | 100 | 100 | 100 | 100 | 100 | 100 | 100 | 100 | 100 | 100 |
| Pachymaran group | 100 | 100 | 100 | 100 | 100 | 100 | 100 | 100 | 100 | 100 | 100 | 100 | 100 | 100 |
| Polysaccharide composition 1 | 100 | 100 | 100 | 100 | 100 | 100 | 100 | 100 | 100 | 100 | 100 | 100 | 100 | 100 |
| Polysaccharide composition 2 | 100 | 100 | 100 | 100 | 100 | 100 | 100 | 100 | 100 | 100 | 100 | 100 | 100 | 100 |
| Polysaccharide composition 3 | 100 | 100 | 100 | 100 | 100 | 100 | 100 | 100 | 100 | 100 | 100 | 100 | 100 | 100 |
| Polysaccharide composition 4 | 100 | 100 | 100 | 100 | 100 | 100 | 100 | 100 | 100 | 100 | 100 | 100 | 100 | 100 |
| Polysaccharide composition 5 | 100 | 100 | 100 | 100 | 100 | 100 | 100 | 100 | 100 | 100 | 100 | 100 | 100 | 100 |
| Polysaccharide composition 6 | 100 | 100 | 100 | 100 | 100 | 100 | 100 | 100 | 100 | 100 | 100 | 100 | 100 | 100 |
| Polysaccharide composition 7 | 100 | 100 | 100 | 100 | 100 | 100 | 100 | 100 | 100 | 100 | 100 | 100 | 100 | 100 |

As was shown in Table 1 and Table 2, after infection by A/PR/8 (H1N1) virus, the mice in blank group did not show obvious abnormal symptoms within 2 days. On Day 3, they started to exhibit tachypnea, beslowinmovement, dull hair, reduced diet, emaciation, etc.. On Day 7, deaths occurred. 5 mice in total died within 2 weeks. The mice in vaccine group did not show obvious morbid symptoms. They had normal diet and slightly less shiny hair color. Their body weights slightly decreased in fluctuation. On Day 10, one mouse died. Mice in all the polysaccharide groups were in good mental state. They had agile action, shiny hair, normal breath, normal diet, naturally increasing body weights, and no death in all the seven groups. Compared to the vaccine group, the polysaccharide composition 1 had a significant better efficacy on weight gain of mice (P<0.05).

**Table 3. Change in body weight after Mexican pandemic flu H1N1 virus infection (n=8, x̅±S)**

| Groups | Change in body weight (%) | | | | | | | | | | | | | |
|---|---|---|---|---|---|---|---|---|---|---|---|---|---|---|
| | Day 1 | Day 2 | Day 3 | Day 4 | Day 5 | Day 6 | Day 7 | Day 8 | Day 9 | Day 10 | Day 11 | Day 12 | Day 13 | Day 14 |
| Blank group | 100 | 99 | 100 | 96 | 94 | 88 | 83 | 81 | 83 | 78 | 76 | 75 | 73 | 71 |
| Vaccine group | 100 | 101 | 102 | 100 | 102 | 99 | 97 | 99 | 98 | 99 | 99 | 99 | 98 | 98 |
| Lentinan group | 100 | 102 | 104 | 105 | 104 | 103 | 102 | 104 | 106 | 107 | 105 | 104 | 106 | 107 |
| Tremellan group | 100 | 101 | 102 | 103 | 104 | 105 | 106 | 109 | 108 | 107 | 106 | 107 | 107 | 107 |
| Pachymaran group | 100 | 102 | 102 | 103 | 104 | 105 | 106 | 106 | 105 | 106 | 107 | 108 | 106 | 107 |
| Polysaccharide composition 1 | 100 | 104 | 105 | 105 | 106 | 106 | 107 | 107 | 108 | 108 | 110 | 110 | 111* | 111* |
| Polysaccharide composition 2 | 100 | 101 | 101 | 102 | 103 | 104 | 101 | 103 | 105 | 106 | 104 | 103 | 105 | 106 |
| Polysaccharide composition 3 | 100 | 101 | 103 | 104 | 103 | 102 | 105 | 108 | 107 | 108 | 109 | 111* | 114* | 116* |
| Polysaccharide composition 4 | 100 | 100 | 101 | 102 | 103 | 104 | 105 | 105 | 104 | 105 | 106 | 107 | 105 | 106 |
| Polysaccharide composition 5 | 100 | 101 | 101 | 102 | 103 | 104 | 101 | 103 | 105 | 106 | 104 | 103 | 105 | 106 |
| Polysaccharide composition 6 | 100 | 101 | 101 | 102 | 103 | 104 | 101 | 103 | 105 | 106 | 104 | 103 | 105 | 106 |
| Polysaccharide composition 7 | 100 | 101 | 103 | 104 | 103 | 102 | 105 | 108 | 107 | 108 | 109 | 111* | 114* | 116* |

| | | | | | | | | | | | | | | |
|---|---|---|---|---|---|---|---|---|---|---|---|---|---|---|
| In comparison with the vaccine group, **P*<0.05, ***P*<0.01. | | | | | | | | | | | | | | |

**Table 4. Comparison of survival rates of mice among various groups after Mexican pandemic flu H1N1 virus infection (n=8)**

| Groups | Survival rates (%) | | | | | | | | | | | | | |
|---|---|---|---|---|---|---|---|---|---|---|---|---|---|---|
| | Day 1 | Day 2 | Day 3 | Day 4 | Day 5 | Day 6 | Day 7 | Day 8 | Day 9 | Day 10 | Day 11 | Day 12 | Day 13 | Day 14 |
| Blank group | 100 | 100 | 100 | 87.5 | 87.5 | 87.5 | 75 | 75 | 62.5 | 62.5 | 50 | 37.5 | 25 | 25 |
| Vaccine group | 100 | 100 | 100 | 100 | 100 | 100 | 100 | 100 | 100 | 87.5 | 87.5 | 87.5 | 75 | 75 |
| Lentinan group | 100 | 100 | 100 | 100 | 100 | 100 | 100 | 100 | 100 | 100 | 100 | 100 | 100 | 100 |
| Tremellan group | 100 | 100 | 100 | 100 | 100 | 100 | 100 | 100 | 100 | 100 | 100 | 100 | 100 | 100 |
| Pachymaran group | 100 | 100 | 100 | 100 | 100 | 100 | 100 | 100 | 100 | 100 | 100 | 100 | 87.5 | 87.5 |
| Polysaccharide composition 1 | 100 | 100 | 100 | 100 | 100 | 100 | 100 | 100 | 100 | 100 | 100 | 100 | 100 | 100 |
| Polysaccharide composition 2 | 100 | 100 | 100 | 100 | 100 | 100 | 100 | 100 | 100 | 100 | 100 | 100 | 100 | 100 |
| Polysaccharide composition 3 | 100 | 100 | 100 | 100 | 100 | 100 | 100 | 100 | 100 | 100 | 100 | 100 | 100 | 100 |
| Polysaccharide composition 4 | 100 | 100 | 100 | 100 | 100 | 100 | 100 | 100 | 100 | 100 | 100 | 100 | 100 | 100 |
| Polysaccharide composition 5 | 100 | 100 | 100 | 100 | 100 | 100 | 100 | 100 | 100 | 100 | 100 | 100 | 100 | 100 |
| Polysaccharide composition 6 | 100 | 100 | 100 | 100 | 100 | 100 | 100 | 100 | 100 | 100 | 100 | 100 | 100 | 100 |
| Polysaccharide composition 7 | 100 | 100 | 100 | 100 | 100 | 100 | 100 | 100 | 100 | 100 | 100 | 100 | 100 | 100 |

As was shown in Table 3 and Table 4, after infection by Mexican pandemic flu H1N1 virus, the mice in blank group started to exhibit tachypnea, beslowinmovement, dull hair, reduced diet, emaciation, etc. On Day 4, deaths occurred. 6 mice in total died within 2 weeks. The mice in vaccine group did not show obvious morbid symptoms. They had normal diet and slightly less shiny hair color. Their body weights slightly decreased in fluctuation. On Day 10, one mouse died, and a total of 2 mice died within 2 weeks. Mice in each polysaccharide group were in good mental state. They had agile action, shiny hair, normal breath, normal diet, and naturally increasing body weights. One mouse in the pachymaran group died on Day 13, and no mouse died in all the other groups. Compared to the vaccine group, polysaccharide compositions 1, 3 and 7 had significantly better efficacy on weight gain of mice (P<0.05).

### Experimental example 2

Test mice in experimental example 1 were infected by influenza virus via nose-dripping.

On Day 35 (Day 3 after infection), 3 mice were taken from each group, from which blood and lungs were collected.

On Day 38 (Day 6 after infection), 3 mice were taken from each group, from which blood and lungs were collected.

On Day 46 (Day 14 after infection), the mice were weighed and sacrificed, from which blood and lungs were collected.

### ①Methods for collecting blood:

Blood was collected by enucleating eyeballs of the mice to allow the blood dripping freely into a 1.5 ml centrifuge tube. The blood was left stand for 2 hours before centrifugation at 3500 rpm for 10 min to obtain the serum, which was divided into aliquots in 200 µl PCR tube and frozen at -20 °C for later use. Antibody titers in the serum were detected by ELISA (the results are as shown in Table 5 and Table 6).

### ②Lung collection

Mice were weighed and sacrificed. The lungs were collected aseptically, weighed and frozen. The lung index was calculated (the results are as shown in Table 7 and Table 8) with the formula lung index = lung weight (mg)/body weight (g).

An amount of PBS was added. The lung tissues were adequately homogenized manually or using a homogenizer. Virus titers in the tissues were detected by dilution method of counting (Table 9 and Table 10).

The homogenate of lung tissues was centrifuged for about 20 min. The supernatant was collected. The contents of IL-2 and IL-5 in the homogenate of lung tissues were detected respectively according to the procedure in the instructions of the ELISA kit for mice cytokines IL-2 and IL-5 (Table 11 and Table 12).

**Table 5. Change of specific antibodies in mice serum before and after infection by A/PR/8 (H1N1) virus (n=3, x̅±S)**

| Groups | Antibody titer (Log2) | | | |
|---|---|---|---|---|
| | Day 0 | Day 3 | Day 6 | Day 14 |
| Blank group | 3.2±0.23 | 3.9±0.31 | 3.8±0.29 | 4.2±0.33 |
| Vaccine group | 3.2±0.25 | 5.6±0.44 | 7.1±0.56 | 7.0±0.55 |
| Lentinan group | 3.3±0.27 | 5.8±0.46 | 7.8±0.69 | 7.7±0.65 |
| Tremellan group | 3.2±0.23 | 6.9±0.54 | 7.7±0.68 | 7.9±0.67 |
| Pachymaran group | 3.1±0.28 | 7.0±0.55 | 8.1±0.70* | 8.0±0.70* |
| Polysaccharide composition 1 | 3.2±0.25 | 8.2±0.65* | 9.2±0.72*^{#△&} | 9.5±0.74*^{#△&} |
| Polysaccharide composition 2 | 3.2±0.35 | 7.0±0.55 | 8.9±0.70* | 9.1±0.70*^{#△} |
| Polysaccharide composition 3 | 3.2±0.15 | 5.8±0.46 | 9.8±0.69*^{#△&} | 9.3±0.65*^{#△&} |
| Polysaccharide composition 4 | 3.2±0.45 | 6.9±0.54 | 8.7±0.68* | 9.1±0.67*^{#△} |
| Polysaccharide composition 5 | 3.2±0.24 | 7.1±0.15 | 8.9±0.60* | 9.1±0.76*^{#△} |
| Polysaccharide composition 6 | 3.2±0.21 | 7.0±0.35 | 9.0±0.73*^{#△&} | 9.1±0.74*^{#△} |
| Polysaccharide composition 7 | 3.2±0.25 | 5.9±0.36 | 9.9±0.79*^{#△&} | 9.9±0.75*^{#△&} |

| | | | | |
|---|---|---|---|---|
| Notes: in comparison with the vaccine group, **P*<0.05; in comparison with the lentinan group, ^{#}*P*<0.05; in comparison with the tremellan group, ^{△}*P*<0.05; in comparison with the pachymaran group, ^{&}*P*<0.05. | | | | |

As was shown in Table 5, before the A/PR/8 (H1N1) virus infection, no specific antibodies were present in the serum of the blank group as negative control. There was no significant difference regarding the levels of specific antibodies in the serum amongst all of the vaccination groups (pure vaccine group and polysaccharide groups). From Day 3 after the virus infection, specific antibodies increased in all the groups except the blank group, wherein the level thereof in the polysaccharide composition 1 group was significantly higher than that in the pure vaccine group (*P*<0.05), and the antibodies in the blank group were always at lower levels.

On Day 6 after infection, as compared to the vaccine group, there were significant differences in the pachymaran group and the 7 groups of polysaccharide compositions (*P* < 0.05). As compared to the lentinan group, tremellan group and pachymaran group, there were significant differences in the polysaccharide compositions 1, 3, 6 and 7 (*P*<0.05).

On Day 14 after infection, as compared to the vaccine group, the pachymaran group and the 7 groups of polysaccharide compositions were capable of significantly increasing the contents of specific antibodies in mice serum (P< 0.05). As compared to the lentinan group and tremellan group, all the 7 groups of polysaccharide compositions had significant effects on increase of specific antibodies in mice serum. As compared to the pachymaran group, there were significant increases in specific antibodies in serum of mice administered with polysaccharide compositions 1, 3 and 7 (*P*<0.05).

The above results suggested that the polysaccharide compositions had significant better efficacies on improvement of decrease in level of antibody induced by infection of A/PR/8 (H1N1) virus than the single polysaccharide groups, wherein the polysaccharide compositions 1, 3 and 7 had better effects.

**Table 6. Change of specific antibodies in mice serum before and after the infection of Mexican pandemic flu H1N1 virus (n=3, x̅±S)**

| Groups | Antibody titer (Log2) | | | |
|---|---|---|---|---|
| | Day 0 | Day 3 | Day 6 | Day 14 |
| Blank group | 4.6±0.36 | 5.6±0.44 | 5.5±0.43 | 6.0±0.48 |
| Vaccine group | 4.6±0.36 | 8.1±0.64 | 10.2±0.81 | 10.1±0.80 |
| Lentinan group | 4.8±0.36 | 8.4±0.66 | 12.9±1.00* | 13.1±0.94* |
| Tremellan group | 4.6±0.33 | 9.9±0.78* | 12.5±0.99* | 12.6±0.98* |
| Pachymaran group | 4.5±0.36 | 10.1±0.80* | 12.8±1.01* | 12.8±1.01* |
| Polysaccharide composition 1 | 4.9±0.36 | 17.7±0.93*^{#△&} | 19.9±1.05*^{#△&} | 20.5±1.08*^{#△&} |
| Polysaccharide composition 2 | 4.9±0.51 | 15.1±0.80*^{#△&} | 19.2±1.01*^{#△&} | 19.2±1.01*^{#△&} |
| Polysaccharide composition 3 | 4.6±0.22 | 11.4±0.66* | 15.7±1.00*^{#△&} | 19.0±0.94*^{#△&} |
| Polysaccharide composition 4 | 4.6±0.65 | 12.9±0.78* | 17.5±0.99*^{#△&} | 18.4±0.98*^{#△&} |
| Polysaccharide composition 5 | 4.8±0.65 | 15.0±0.78*^{#△&} | 19.0±0.98*^{#△&} | 19.0±0.97*^{#△&} |
| Polysaccharide composition 6 | 4.5±0.79 | 15.4+0.96*^{#△&} | 19.6±1.21*^{#△&} | 19.6±1.19*^{#△&} |
| Polysaccharide composition 7 | 4.7±0.51 | 13.4±0.62* | 18.8±0.78*^{#△&} | 17.1±0.77*^{#△&} |

| | | | | |
|---|---|---|---|---|
| Notes: in comparison with the vaccine group, **P*<0.05; in comparison with the lentinan group, ^{#}*P*<0.05; in comparison with the tremellan group, ^{△}*P*<0.05; in comparison with the pachymaran group, ^{&}*P*<0.05. | | | | |

As was shown in Table 6, before the Mexican pandemic flu H1N1 virus infection, no specific antibodies were present in the serum of the blank group as negative control. There was no significant difference regarding the levels of specific antibodies in the serum amongst all of the vaccination groups (pure vaccine group and polysaccharide groups). From Day 3 after the virus infection, specific antibodies significantly increased in all the groups. The levels of specific antibodies in all the groups except the lentinan group were significantly higher than that in the pure vaccine group (*P*<0.05). The antibodies in the blank group were always at lower levels. As compared to the single polysaccharide groups, the levels of antibodies for the polysaccharide compositions 1, 2, 5 and 6 were significantly higher (*P*<0.05).

On Day 6 and Day 14 after infection, as compared to the vaccine group, the levels of antibodies for all the groups of polysaccharide compositions significantly increased (*P*<0.05). As compared to the single polysaccharide groups, the levels of antibodies for all the groups of polysaccharide compositions significantly increased (*P*<0.05)

The above results suggested that all the polysaccharide compositions had significant better efficacies on improvement of decrease in level of antibody induced by infection of Mexican pandemic flu H1N1 virus than the single polysaccharide groups.

**Table 7. Change in mice lung index before and after infection of A/PR/8 (H1N1) virus (n=3, x̅±S)**

| Groups | Lung index (mg/g) | | | |
|---|---|---|---|---|
| | Day 0 | Day 3 | Day 6 | Day 14 |
| Blank group | 7.18±0.14 | 9.81±0.77 | 17.89±1.89 | 17.49±1.49 |
| Vaccine group | 7.17±0.13 | 8.51±0.97 | 12.00±0.89 | 12.23±0.78 |
| Lentinan group | 7.09±0.05 | 8.22±1.18 | 8.98±0.90* | 8.88±0.80* |
| Tremellan group | 7.88±0.84 | 8.14±1.10 | 8.99±0.91* | 8.91±0.83* |
| Pachymaran group | 7.80±0.76 | 8.23±1.19 | 8.23±1.15* | 8.21±1.13* |
| Polysaccharide composition 1 | 7.90±0.86 | 8.01±0.97 | 7.27±1.59*^{#△ &} | 7.51±1.49*^{#△ &} |
| Polysaccharide composition 2 | 7.80±0.76 | 7.26±0.22* | 7.89±1.81*^{#△} | 7.29±1.71*^{#△ &} |
| Polysaccharide composition 3 | 7.09±0.05 | 7.89±0.85 | 7.27±1.59*^{#△ &} | 7.53±1.49*^{#△ &} |
| Polysaccharide composition 4 | 7.88±0.84 | 7.67±0.63 | 7.96±1.88*^{#△} | 7.46±1.38*^{#△ &} |
| Polysaccharide composition 5 | 7.81±0.56 | 7.36±0.21* | 7.99±0.91*^{#△} | 7.09±1.92*^{#△ &} |
| Polysaccharide composition 6 | 7.80±0.66 | 7.32±0.32* | 7.19±1.23*^{#△ &} | 7.11±0.99*^{#△ &} |
| Polysaccharide composition 7 | 7.09±0.05 | 7.99±0.75 | 7.22±0.99*^{#△ &} | 7.43±0.45*^{#△ &} |

| | | | | |
|---|---|---|---|---|
| Notes: in comparison with the vaccine group, **P*<0.05; in comparison with the lentinan group, ^{#}*P*<0.05; in comparison with the tremellan group, ^{△}*P*<0.05; in comparison with the pachymaran group, ^{&}*P*<0.05. | | | | |

It was observed morphologically that, in the infected mice in blank group, lung volumes significantly increased. Large areas of lung consolidation showing dark red appearance were observed throughout the lung. In the mice in vaccine group, the entire lung appeared reddish, and the lesion degree was obviously slighter than that of the blank group, with some dark red lesions of lung consolidation observed. In the infected mice in polysaccharide groups, the surface of lung appeared reddish with no ecchymosis or petechiae, had no obvious areas of lung consolidation, and very few lung lobes exhibited the phenomena of congestion.

The results of lung index were as shown in Table 7. Starting from Day 3, the lung indexes of the mice in the blank group significantly increased. The lung indexes of the mice in other groups were relatively stable. As compared to the vaccine group, the polysaccharide compositions 2, 5 and 6 showed the effects of significantly improving lung indexes (*P*<0.05).

On Day 6 after infection, as compared to the vaccine group, all the polysaccharide groups showed effects of significantly improving the lung indexes (P < 0.05). As compared to the lentinan group and tremellan group, all the polysaccharide compositions had significantly better improving effects on mice lung index than lentinan and tremellan (*P*<0.05). As compared to pachymaran, the lung indexes of mice in polysaccharide compositions 1, 3, 6 and 7 significantly decreased (*P*<0.05).

On Day 14 after infection, as compared to the vaccine group, all the polysaccharide groups showed effects of significantly improving the lung indexes (*P*<0.05). As compared to the single polysaccharide groups, all the 7 groups of polysaccharide compositions can reduce the lung index of mice (*P*<0.05).

The above results suggested that polysaccharide compositions with different ratios had significantly better efficacies on improving lung damage induced by infection of A/PR/8 (H1N1) virus than the single polysaccharides.

**Table 8. Change in mice lung index before and after the infection of Mexican pandemic flu H1N1 virus (n=3, x̅±S)**

| Groups | Lung index ( mg/g ) | | | |
|---|---|---|---|---|
| | Day 0 | Day 3 | Day 6 | Day 14 |
| Blank group | 10.32±0.20 | 14.10±1.11 | 25.72±2.72 | 25.14±2.14 |
| Vaccine group | 10.31±0.19 | 11.51±1.39 | 17.25±1.28 | 17.58±1.12 |
| Lentinan group | 10.19±0.07 | 11.82±1.70 | 12.43±1.29* | 12.89±1.15* |
| Tremellan group | 11.33±1.21 | 11.7±1.58 | 12.45±1.31* | 12.93±1.19* |
| Pachymaran group | 11.21±1.09 | 11.83±1.71 | 12.39±1.65* | 12.36±1.62* |
| Polysaccharide composition 1 | 11.36±1.24 | 11.51±1.39 | 11.03±2.29*^{#△ &} | 10.88±2.14*^{#△ &} |
| Polysaccharide composition 2 | 11.21±1.09 | 10.44±0.32 | 11.34±2.60*^{#△ &} | 11.20±2.46*^{#△ &} |
| Polysaccharide composition 3 | 10.19±0.07 | 11.34±1.22 | 11.03±2.29*^{#△ &} | 10.88±2.14*^{#△ &} |
| Polysaccharide composition 4 | 11.33±1.21 | 11.03±0.91 | 11,44±2.70*^{#△ &} | 10.72±1.98*^{#△ &} |
| Polysaccharide composition 5 | 11.10±1.23 | 10.33±0.92 | 11.23±2.76*^{#△ &} | 11.09±2.02*^{#△ &} |
| Polysaccharide composition 6 | 10.99±1.06 | 10.23±0.80 | 11.12:t2.38*^{#△ &} | 10.97±1.75*^{#△ &} |
| Polysaccharide composition 7 | 10.29±1.20 | 11.46±0.90 | 11.14±2.68*^{#△ &} | 10.99±1.96*^{#△ &} |

| | | | | |
|---|---|---|---|---|
| Notes: in comparison with the vaccine group, **P*<0.05; in comparison with the lentinan group, ^{#}*P*<0.05; in comparison with the tremellan group, ^{△}*P*<0.05; in comparison with the pachymaran group, ^{&}*P*<0.05. | | | | |

It was observed morphologically that, in the infected mice in blank group, lung volumes significantly increased. Large areas of lung consolidation showing dark red appearance were observed throughout the lung. In the mice in vaccine group, the entire lung appeared reddish, and the lesion degree was obviously slighter than that of the blank group, with some dark red lesions of lung consolidation observed. In the infected mice in polysaccharide groups, the surface of lung appeared reddish with no ecchymosis or petechiae, had no obvious areas of lung consolidation, and very few lung lobes exhibited the phenomena of congestion.

The results of lung index were as shown in Table 8. Starting from Day 3, the lung indexes of the mice in the blank group significantly increased. The lung indexes of the mice in other groups were relatively stable.

On Day 6 and Day 14, as compared to the vaccine group, all the polysaccharide groups showed effects of significantly reducing the lung index (P< 0.05). As compared to the single polysaccharide groups, the lung indexes of mice for all polysaccharide composition groups with 7 ratios were significantly lower (P <0.05).

The above results suggested that polysaccharide compositions with different ratios had significantly better efficacies on improving lung damage induced by infection of Mexican pandemic flu H1N1 virus than single polysaccharides.

**Table 9. Change in content of H1N1 virus in the lung of mice after the infection of A/PR/8 (H1N1) virus (n=3, x̅±S)**

| Groups | Viral titer ( O.D. ) | | |
|---|---|---|---|
| | Day 3 | Day 6 | Day 14 |
| Blank group | 0.46±0.03 | 0.21±0.02 | 0.21±0.03 |
| Vaccine group | 0.15±0.01 | 0.17±0.01 | 0.19±0.01 |
| Lentinan group | 0.12±0.01 | 0.13±0.03 | 0.14±0.02* |
| Tremellan group | 0.13±0.01 | 0.13±0.02 | 0.15±0.01* |
| Pachymaran group | 0.13±0.01 | 0.12±0.01 | 0.14±0.01* |
| Polysaccharide composition 1 | 0.09±0.01* | 0.08±0.03*^{#△ &} | 0.10±0.01* |
| Polysaccharide composition 2 | 0.13±0.01 | 0.11±0.01*^{#△ &} | 0.12±0.02* |
| Polysaccharide composition 3 | 0.11±0.01* | 0.09±0.02*^{#△ &} | 0.11±0.04* |
| Polysaccharide composition 4 | 0.13±0.01 | 0.11±0.01*^{#4 &} | 0.15±0.03* |
| Polysaccharide composition 5 | 0.12±0.02 | 0.11±0.04*^{#△ &} | 0.11±0.06* |
| Polysaccharide composition 6 | 0.13±0.03 | 0.11±0.06*^{#△ &} | 0.15±0.02* |
| Polysaccharide composition 7 | 0.10±0.02* | 0.09±0.01*^{#△ &} | 0.10±0.04* |

| | | | |
|---|---|---|---|
| Notes: in comparison with the vaccine group, ^{*}*P*<0.05. | | | |

As was shown in Table 9, starting from Day 3 after the infection of A/PR/8 (H1N1) virus, the A/PR/8 (H1N1) viral titer of the lung of the mice in the blank group reached very high level, while the A/PR/8 (H1N1) viral titers of the lung of the mice in the vaccine group and polysaccharide groups were significantly lower than that in the blank group (*P*<0.05), wherein the A/PR/8 (H1N1) viral titers of the lung of the mice in the groups of polysaccharide compositions 1, 3 and 7 were lower than that of the vaccine group (*P*<0.05).

On Day 6 after infection, the contents of A/PR/8 (H1N1) virus in the lung of all the groups of polysaccharide compositions was significantly lower than that of the vaccine group (*P*<0.05); as compared to the single polysaccharide group, all the 7 groups of polysaccharide composition can reduce the content of A/PR/8 (H1N1) virus in the lung of mice (*P*<0.05).

On Day 14 after infection, the contents of A/PR/8 (H1N1) virus in the lung of mice in all of the polysaccharide groups were significantly lower than that of the vaccine group (*P*<0.05). There was no difference between the effects of the polysaccharide compositions and the single polysaccharides.

**Table 10. Change in content of H1N1 virus in the lung of mice after infection of Mexican pandemic flu H1N1 virus (n=3, x̅±S)**

| Groups | Viral titer (O.D.) | | |
|---|---|---|---|
| | Day 3 | Day 6 | Day 14 |
| Blank group | 0.36±0.05 | 0.27±0.05 | 0.21±0.02 |
| Vaccine group | 0.28±0.02 | 0.22±0.02 | 0.20±0.02 |
| Lentinan group | 0.19±0.02 | 0.18±0.01 | 0.14±0.01* |
| Tremellan group | 0.22±0.02 | 0.19±0.02 | 0.16±0.01* |
| Pachymaran group | 0.18±0.01 | 0.20±0.01 | 0.15±0.01* |
| Polysaccharide composition 1 | 0.15±0.01*^{#△ &} | 0.13±0.01*^{#△ &} | 0.12±0.01* |
| Polysaccharide composition 2 | 0.18±0.01 | 0.14±0.01*^{#△ &} | 0.15±0.01* |
| Polysaccharide composition 3 | 0.19±0.02 | 0.11±0.01*^{#△ &} | 0.12±0.01* |
| Polysaccharide composition 4 | 0.22±0.02 | 0.15±0.02*^{#△ &} | 0.16±0.01* |
| Polysaccharide composition 5 | 0.17±0.02* | 0.13±0.01*^{#△ &} | 0.14±0.01* |
| Polysaccharide composition 6 | 0.18±0.02 | 0.15±0.02*^{#△ &} | 0.15±0.02* |
| Polysaccharide composition 7 | 0.19±0.01 | 0.15±0.01*^{#△ &} | 0.16±0.01* |

| | | | |
|---|---|---|---|
| Notes: in comparison with the vaccine group, **P*<0.05; in comparison with the lentinan group, ^{#}*P*<0.05; in comparison with the tremellan group, ^{△}*P*<0.05; in comparison with the pachymaran group, ^{&}*P*<0.05. | | | |

As was shown in Table 10, starting from Day 3 after the infection of Mexican pandemic flu H1N1 virus, the Mexican pandemic flu H1N1 viral titer in the blank group reached very high level, while the Mexican pandemic flu H1N1 viral titers in the vaccine group and polysaccharide groups were significantly lower than that in the blank group (*P*<0.05), wherein the Mexican pandemic flu H1N1 viral titers in the groups of polysaccharide compositions 1 and 5 were lower than that in the vaccine group (*P*<0.05). As compared to the single polysaccharide group, the polysaccharide composition 1 showed the effects of significantly reducing the viral titer (*P*<0.05).

On Day 6 after infection, the Mexican pandemic flu H1N1 viral titers in the lungs of the mice in all groups of the polysaccharide compositions were significantly lower than that in the vaccine group (*P*<0.05). As compared to the single polysaccharide groups, the contents of Mexican pandemic flu H1N1 virus in the lung of mice of all the groups of the polysaccharide compositions were significantly reduced (*P*<0.05).

On Day 14 after infection, the contents of Mexican pandemic flu H1N1 virus in the lung of all the groups were in relatively normal levels. The Mexican pandemic flu H1N1 viral titers of mice in all the polysaccharide groups were significantly lower than that in the vaccine group (*P*<0.05).

**Table 11. Change in contents of IL-2 and IL-5 in the lung of mice before and after the infection of A/PR/8 (H1N1) virus (n=3, x̅±S)**

| Indicators | Groups | Day 0 | Day 3 | Day 6 | Day 14 |
|---|---|---|---|---|---|
| IL-2 content (unit: pg/ml) | Blank group | 3.08±0.06 | 3.64±0.32 | 23.54±2.11 | 73.78±6.64 |
| | Vaccine group | 4.08±0.08 | 4.64±0.41 | 64.61±3.11 | 124.85±9.43 |
| | Lentinan group | 6.08±0.12 | 11.64±0.59 | 76.64±1.49 | 146.88±8.71 |
| | Tremellan group | 7.04±0.14 | 7.60±0.68 | 79.16±7.12 | 132.61±13.46 |
| | Pachymaran group | 7.94±0.15 | 13.30±1.19 | 74.34±6.69 | 144.79±13.03 |
| | Polysaccharide composition 1 | 8.34±0.16 | 13.70±1.23 | 73.17±6.58 | 143.62±12.92 |
| | Polysaccharide composition 2 | 8.44±0.16 | 13.80±1.24 | 74.8±6.73 | 145.25±13.07 |
| | Polysaccharide composition 3 | 9.24±0.16* | 14.60±1.22*^{△} | 75.63±6.62* | 154.08±12.96*^{△} |
| | Polysaccharide composition 4 | 8.44±0.16 | 13.80±1.24 | 73.58±6.62 | 144.03±12.96 |
| | Polysaccharide composition 5 | 8.54±0.18 | 13.77±1.04 | 74.83±6.63 | 143.15±10.17 |
| | Polysaccharide composition 6 | 8.44±0.17 | 13.41±0.94 | 73.48±6.09 | 149.25±14.34 |
| | Polysaccharide composition 7 | 9.34±0.26* | 14.70±1.23*^{△} | 77.21±5.12* | 159.08±11.76*^{△} |
| IL-5 content (unit: pg/ml) | Blank group | 17.29±1.53 | 19.85±1.76 | 44.33±3.94 | 55.56±4.94 |
| | Vaccine group | 14.29±1.27 | 20.85±1.85 | 55.40±4.93 | 56.63±5.04 |
| | Lentinan group | 16.29±1.44 | 22.85±2.03 | 37.43±3.33 | 69.6±4.33* |
| | Tremellan group | 17.25±1.53 | 23.81±2.11 | 58.37±5.19 | 61.4±6.19 |
| | Pachymaran group | 18.15±1.61 | 29.51±2.62 | 51.55±4.76 | 59.78±5.76 |
| | Polysaccharide composition 1 | 18.55±1.65 | 39.91±2.66* | 62.38±4.66* | 73.61±5.66* |
| | Polysaccharide composition 2 | 18.65±1.65 | 30.01±2.67 | 54.01±4.80 | 71.24±5.80* |
| | Polysaccharide composition 3 | 21.45±1.64* | 39.81±2.65* | 62.84±4.70*^{&} | 74.07±5.70*^{&} |
| | Polysaccharide composition 4 | 18.65±1.65 | 30.01±2.67 | 52.79±4.69 | 72.02±5.69* |
| | Polysaccharide composition 5 | 18.73±1.05 | 29.01±2.47 | 56.21±4.44 | 70.44±5.81* |
| | Polysaccharide composition 6 | 18.12±3.15 | 29.32±1.61 | 57.06±3.56 | 70.56±4.80* |
| | Polysaccharide composition 7 | 22.45±1.44* | 39.89±3.65* | 63.14±5.66*^{&} | 75.17±4.70*^{&} |

| | | | | | |
|---|---|---|---|---|---|
| Notes: in comparison with the vaccine group, **P*<0.05; in comparison with the tremellan group, ^{△}*P*<0.05; in comparison with the pachymaran group, ^{&}*P*<0.05. | | | | | |

After the mice were infected by A/PR/8 (H1N1) virus, the levels of cytokines in the homogenate of lung significantly changed. The IL-2 gradually increased as the time of infection increased. As can be known from Table 11, with respect to improvement of the level of IL-2 in the homogenate of lung, the effects of the groups of polysaccharide compositions 3 and 7 were significantly better than those of the vaccine group and tremellan group (P < 0.05), suggesting that the polysaccharide compositions 3 and 7 can play an anti-virus role by improving the IL-2 level in the homogenate of lung, and the effects were significantly better than those of the single polysaccharide groups.

After the mice were infected by A/PR/8 (H1N1) virus, the cytokine IL-5 in the lung gradually increased as the time of infection increased. On Day 3 and Day 6 of the virus infection, the IL-5 level in the lung of mice of the groups of polysaccharide compositions 1, 3 and 7 significantly increased as compared to the vaccine group. On Day 14, the IL-5 level in the lung of mice in the lentinan group and all the groups of the polysaccharide compositions significantly increased as compared to the vaccine group. Starting from Day 6, the effects of polysaccharide compositions 3 and 7 were significantly better than that of the pachymaran. The above results suggested that the lentinan and the polysaccharide compositions can play an anti-virus role by improving the IL-5 level in the homogenate of lung, wherein some polysaccharide compositions had better efficacies than the single polysaccharides.

**Table 12. Change in contents of IL-2 and IL-5 in the lung of mice before and after infection of Mexican pandemic flu H1N1 virus (n=3, x̅±S)**

| Indicators | Groups | Day 0 | Day 3 | Day 6 | Day 14 |
|---|---|---|---|---|---|
| IL-2 content (unit: pg/ml) | Blank group | 5.21±0.08 | 10.98±0.45 | 72.21±2.90 | 100.97±9.09 |
| | Vaccine group | 7.58±0.11 | 12.35±0.57 | 87.36±4.26 | 143.49±12.91 |
| | Lentinan group | 8.32±0.17 | 11.09±0.82 | 102.77±2.05 | 182.58±11.93** |
| | Tremellan group | 9.63±0.19 | 10.40±0.94 | 108.33±9.75* | 194.74±18.43** |
| | Pachymaran group | 10.87±0.22 | 18.20±1.64 | 101.73±9.16 | 198.15±17.83** |
| | Polysaccharide composition 1 | 11.41±0.23* | 18.75±1.69 | 100.13±9.01 | 196.54±17.69** |
| | Polysaccharide composition 2 | 11.55±0.23* | 18.89±1.70* | 102.36±9.21 | 198.77±17.89** |
| | Polysaccharide composition 3 | 11.28±0.23 | 18.61±1.68 | 100.76±9.07 | 197.17±17.75** |
| | Polysaccharide composition 4 | 11.05±0.23 | 18.89±1.70* | 100.69±9.06 | 197.11±17.74** |
| | Polysaccharide composition 5 | 11.13±0.23* | 18.70±1.67 | 101.34±8.88 | 196.79±17.38** |
| | Polysaccharide composition 6 | 11.67±0.20* | 19.07±1.50* | 103.39±7.97* | 204.76±15.61** # |
| | Polysaccharide composition 7 | 11.50±0.21* | 18.98±1.58* | 102.78±8.43 | 201.12±16.50** # |
| IL-5 content (unit: pg/ml) | Blank group | 23.04±2.05 | 26.46±2.35 | 59.08±5.26 | 74.05±6.59 |
| | Vaccine group | 19.05±1.70 | 27.79±2.47 | 73.84±6.57 | 75.48±6.72 |
| | Lentinan group | 21.71±1.93 | 30.45±2.71 | 49.89±4.44 | 84.85±5.77 |
| | Tremellan group | 22.99±2.05 | 31.73±2.82 | 77.8±6.92* | 92.76±8.26* |
| | Pachymaran group | 24.19±2.15 | 39.33±3.50 | 71.37±6.35 | 86.34±7.68 |
| | Polysaccharide composition 1 | 24.72±2.20 | 39.86±3.55 | 69.81±6.21 | 94.78±7.55 |
| | Polysaccharide composition 2 | 24.86±2.21 | 40.00±3.56 | 71.98±6.41 | 96.95±7.74 |
| | Polysaccharide composition 3 | 24.59±2.19 | 39.73±3.54 | 70.43±6.27 | 95.39±7.60 |
| | Polysaccharide composition 4 | 24.86±2.21 | 40.00±3.56 | 70.36±6.26 | 95.33±7.59 |
| | Polysaccharide composition 5 | 24.61±2.17 | 39.60±3.49 | 71.26±6.14 | 96.08±7.44 |
| | Polysaccharide composition 6 | 25.11±1.95 | 40.40±3.13 | 72.70±5.51 | 97.82±6.68*^{#&} |
| | Polysaccharide composition 7 | 25.08±2.06 | 40.53±3.31 | 71.83±5.82 | 97.10±7.06*^{#&} |

| | | | | | |
|---|---|---|---|---|---|
| Notes: in comparison with the vaccine group, **P*<0.05, ***P*<0.01; in comparison with the lentinan group, ^{#}*P*<0.05; in comparison with the pachymaran group, ^{&}*P*< 0.05. | | | | | |

After the mice were infected by Mexican pandemic flu H1N1 virus, the levels of cytokines in the homogenate of lung significantly changed, and the IL-2 gradually increased as the time of infection increased. As can be known from Table 12, with respect to improvement to the level of IL-2 in the homogenate of lung, the effects of all of the polysaccharide groups were significantly better than that of the vaccine group (*P*<0.05). As compared to the lentinan group, the effects of polysaccharide compositions 6 and 7 were all significantly better than that of the lentinan. This suggested that, with respect of improving the IL-2 level in the homogenate of lung, the effects of the polysaccharide compositions were better than that of single polysaccharide.

After the mice were infected by Mexican pandemic flu H1N1 virus, the cytokine IL-5 in the lung gradually increased as the time of infection increased. On Day 3 of the virus infection, there was no significant difference amongst all of the groups. On Day 6 and Day 14, the IL-5 level in the lung of mice in the tremellan group, the groups of polysaccharide compositions 6 and 7 significantly increased as compared to the vaccine group. As compared to the lentinan and pachymaran, the polysaccharide compositions 6 and 7 were significantly better. This suggested that, with respect to improving the IL-5 level in the homogenate of lung, the effects of the polysaccharide compositions were better than that of single polysaccharide.

## Claims

1. A polysaccharide composition consisting of lentinan, pachymaran and tremella polysaccharide;
wherein the mass ratio of lentinan, pachymaran and tremella polysaccharide is (1-3):(1-3):(1-3).

2. The polysaccharide composition according to claim 1, wherein the mass ratio of lentinan, pachymaran and tremella polysaccharide is (1-3):(1-2):(1-2).

3. The polysaccharide composition according to claim 1, wherein the mass ratio of lentinan, pachymaran and tremella polysaccharide is (2-3):1:1.

4. The polysaccharide composition according to claim 1, wherein the mass ratio of lentinan, pachymaran and tremella polysaccharide is (1-2):1:1.

5. The polysaccharide composition according to claim 1, wherein the mass ratio of lentinan, pachymaran and tremella polysaccharide is 1:(1-3):(1-3).

6. The polysaccharide composition of any of claims 1 to 5 for use as a medicament.

7. The polysaccharide composition of any of claims 1 to 5 for use as an antiinfluenza virus medicament.

8. The polysaccharide composition of any of claims 1 to 5 for use in the treatment of influenza.

9. The polysaccharide composition for use according to claim 7 or 8, wherein the influenza virus is seasonal influenza virus or influenza A virus.

10. The polysaccharide composition for use according to claim 7 or 8, wherein the human dosage of the polysaccharide composition for combating influenza virus is 0.1-1 g/day.

11. Use of the polysaccharide composition of any of claims 1 to 5 as a food.

## Patentansprüche

1. Polysaccharidzusammensetzung, umfassend Lentinan-, Pachymaran- und Tremella-Polysaccharid;
wobei das Masseverhältnis von Lentinan-, Pachymaran- und Tremella-Polysaccharid (1-3):(1-3) : (1-3) ist.

2. Polysaccharidzusammensetzung nach Anspruch 1, wobei das Masseverhältnis von Lentinan-, Pachymaran- und Tremella-Polysaccharid (1-3):(1-2):(1-2) ist.

3. Polysaccharidzusammensetzung nach Anspruch 1, wobei das Masseverhältnis von Lentinan-, Pachymaran- und Tremella-Polysaccharid (2-3):1:1 ist.

4. Polysaccharidzusammensetzung nach Anspruch 1, wobei das Masseverhältnis von Lentinan-, Pachymaran- und Tremella-Polysaccharid (1-2):1:1 ist.

5. Polysaccharidzusammensetzung nach Anspruch 1, wobei das Masseverhältnis von Lentinan-, Pachymaran- und Tremella-Polysaccharid 1:(1-3):(1-3) ist.

6. Polysaccharidzusammensetzung nach einem der Ansprüche 1 bis 5 zur Verwendung als Arzneimittel.

7. Polysaccharidzusammensetzung nach einem der Ansprüche 1 bis 5 zur Verwendung als Arzneimittel gegen Influenzavirus.

8. Polysaccharidzusammensetzung nach einem der Ansprüche 1 bis 5 zur Verwendung bei der Behandlung von Influenza.

9. Polysaccharid¬izusammensetzung zur Verwendung gemäß Anspruch 7 oder 8, wobei es sich bei dem Influenzavirus um saisonales Influenzavirus oder Influenza-A-Virus handelt.

10. Polysaccharid¬izusammensetzung zur Verwendung gemäß Anspruch 7 oder 8, wobei die Dosierung der Polysaccharid¬izusammensetzung zur Bekämpfung von Influenzavirus beim Menschen bei 0,1-1 g/Tag liegt.

11. Verwendung der Polysaccharid¬izusammensetzung nach einem der Ansprüche 1 bis 5 zur Verwendung als Arzneimittel als Lebensmittel.

## Revendications

1. Composition de polysaccharide constituée de polysaccharide de type lentinane, pachymarane et de tremella ;
le rapport en masse de polysaccharide de type lentinane, pachymarane et de tremella étant (1 à 3) : (1 à 3) : (1 à 3) .

2. Composition de polysaccharide selon la revendication 1, le rapport en masse de polysaccharide de type lentinane, pachymarane et de tremella étant (1 à 3) : (1 à 2) : (1 à 2).

3. Composition de polysaccharide selon la revendication 1, le rapport en masse de polysaccharide de type lentinane, pachymarane et de tremella étant (2 à 3) : 1 : 1.

4. Composition de polysaccharide selon la revendication 1, le rapport en masse de polysaccharide de type lentinane, pachymarane et de tremella étant (1 à 2) : 1 : 1.

5. Composition de polysaccharide selon la revendication 1, le rapport en masse de polysaccharide de type lentinane, pachymarane et de tremella étant 1 : (1 à 3) : (1 à 3).

6. Composition de polysaccharide selon l'une quelconque des revendications 1 à 5 pour une utilisation en tant que médicament.

7. Composition de polysaccharide selon l'une quelconque des revendications 1 à 5 pour une utilisation en tant que médicament anti-virus de la grippe.

8. Composition de polysaccharide selon l'une quelconque des revendications 1 à 5 pour une utilisation dans le traitement de la grippe.

9. Composition de polysaccharide pour une utilisation selon la revendication 7 ou 8, le virus de la grippe étant un virus de la grippe saisonnière ou un virus de la grippe A.

10. Composition de polysaccharide pour une utilisation selon la revendication 7 ou 8, le dosage pour un humain de la composition de polysaccharide pour la lutte contre un virus de la grippe étant de 0,1 à 1 g/jour.

11. Utilisation de la composition de polysaccharide selon l'une quelconque des revendications 1 à 5 en tant que produit alimentaire.
